# EUROPÄISCHE PATENTANMELDUNG

(11) **EP 0 526 920 A1**
(43) Veröffentlichungstag der Anmeldung: **10.02.1993**
(21) Anmeldenummer: 92201287.7
(22) Anmeldetag: 07.05.1992
(51) Int. Cl.: F04B 43/00, A61M 5/142

(54) **Pumpenschlauch für eine peristaltische Pumpe**

(30) Priorität: 07.08.1991 DE 4126087
(71) Anmelder: B. Braun Melsungen AG, D-34209 Melsungen (DE)
(72) Erfinder: Knuth, Reinhard, W-3508 Melsungen (DE)
(74) Vertreter: Selting, Günther, Dipl.-Ing.

(57) **Zusammenfassung**

Bei einem Pumpenschlauch (10) für eine peristaltische Pumpe ist es wichtig, daß er mit in bezug auf die Saugseite und die Druckseite der Pumpe korrekter Fließrichtung problemlos in der Pumpe befestigbar ist und sich durch gleichmäßiges Förderverhalten auszeichnet.

Ein solcher Pumpenschlauch (10), der an seinem saugseitigen und an seinem druckseitigen Ende mit einer einstückig angeformten Muffe (12,14) versehen und durch Halteteile an der Pumpe (40) hängend befestigbar ist,
ist dadurch gekennzeichnet, daß
an jeder Muffe (12,14) eine Aufhängevorrichtung (20,21) in Form von zwei symmetrisch zur Längsachse des Schlauches (10) nach entgegengesetzten Seiten gerichteten Laschen (22,23) angeordnet ist, in denen je eine Lochöse (24,25) ausgebildet ist,
daß die Lochösen (24) in den Laschen (22) am saugseitigen Ende des Schlauches (10) eine andere Form und/oder Größe als die Lochösen (25) in den Laschen (23) am druckseitigen Ende des Schlauches (10) haben, und
daß die Lochösen (24,25) auf angepaßte Haltestifte (35,36) der Pumpe (40) aufsteckbar sind.

## Beschreibung

Die Erfindung bezieht sich auf einen Pumpenschlauch für eine peristaltische Pumpe, der an seinem saugseitigen und an seinem druckseitigen Ende mit einer einstückig angeformten Muffe versehen und durch Halteteile an der Pumpe hängend befestigbar ist.

Im medizinischen Bereich werden Peristaltikpumpen oder Schlauchpumpen als Infusionspumpen verwendet. Solche Pumpen ermöglichen den sterilen Transport von größeren Mengen Infusionsflüssigkeit. Der auswechselbare Pumpenschlauch stellt ein preiswertes Einmalerzeugnis dar, welches in steriler Form mit angesetzten Zu- und Ableitungsschläuchen einer Infusionsleitung geliefert wird, vom Benutzer in die Pumpe eingesetzt werden kann und nach Gebrauch fortgeworfen wird. Allerdings werden zur Erzielung gleichbleibender Fördergenauigkeit an den Pumpenschlauch hohe Anforderungen bezüglich Walkfähigkeit, Elastizität, Abriebfestigkeit und Maßhaltigkeit in Längs- und Querrichtung gestellt. Das Fördervolumen hängt von den Querschnittsabmessungen des Pumpenschlauches ab und von dessen Rückstellfähigkeit. Das verwendete Material muß physiologisch unbedenklich, d.h., gegenüber den verwendeten Medien inert sein. Umgebungseinflüsse, wie Temperatur, Luftfeuchte, Licht und Desinfektionsmittel dürfen das Material nicht beeinflußen.

Üblicherweise werden Pumpenschläuche für peristaltische Pumpen aus hochelastischem Material, z.B. Silikon, mit hoher Maßhaltigkeit hergestellt. Mit Hilfe separater spezieller Zwischenstücke, z.B. gemäß DE-GM 84 06 203, sind die Zuleitungs- und Ableitungsschlauchenden der Infusionsleitung werksseitig mit dem Pumpenschlauch verbunden worden und der Benutzer legt den Pumpenschlauch in einen Kanal der peristaltischen Pumpe ein und befestigt die Zwischenstücke an Halterungen des Pumpengehäuses zur lagegerechten Fixierung des Pumpenschlauches. Der Pumpenschlauch verläuft zwischen einem Gegenlager und Pumpenschiebern, die Hubbewegungen gegen den Schlauch ausführen und ihn peristaltisch zusammendrücken. Da das eine Ende der Infusionsleitung mit einem Infusionsflüssigkeitsbehälter oder dergleichen verbunden ist und das andere Ende der Infusionsleitung zum Patienten führt, ist es bei der Einlegung des Pumpenschlauches in die Peristaltikpumpe wichtig, daß die Fließrichtung stimmt, d.h., daß die Infusionsbehälterseite an der Saugseite der Pumpe liegt und die Patientenseite sich an der Druckseite befindet. Dies ist bisher nicht einfach erkennbar, weil die Muffen, die mit den Zwischenstücken zusammengesteckt sind, etwa gleich aussehen und auch die Zwischenstücke nicht ohne weiteres Rückschlüsse auf ihre Zuordnung zu den oberen bzw. unteren Halterungen an dem Kanal des Pumpengehäuses zulassen.

Durch die Weiterentwicklung der medizinischen Kunst steigen die Anforderungen an die Einhaltung bestimmter Grenzwerte bei der Infusionstechnik ständig an. Gleichzeitig ist durch die Vielfalt der Geräte und die hohe Belastung des Bedienungspersonals die Notwendigkeit einer einfachen Erkenn- und Bedienbarkeit weiter gestiegen. Da eine Fehlbedienung letztlich zu einer Gefährdung der Patienten führen kann, ist das eindeutige Fixieren des Pumpenschlauches in der medizinischen Technik zu einer Frage der Sicherheit geworden. Die Verwendung der erwähnten Zwischenstücke, mit denen die Muffen des Pumpenschlauches mit den Zu- und Ableitungsschläuchen zusammengesteckt sind und die selbst an dem Pumpengehäuse befestigt werden müssen, hat den Nachteil, daß Schlauchdurchmesser und Schlauchlänge infolge von Dehnungen oder Stauchungen sowie die Lage des Pumpenschlauches in der Pumpe von der jeweiligen Montage abhängig sind, so daß eine Förderrate bzw. Infusionsrate nicht genau eingehalten werden kann, sondern von Pumpenschlauch zu Pumpenschlauch in verhältnismäßig großem Toleranzbereich schwankt.

Ein Pumpenschlauch mit einstückig angeformten Muffen ist in DE 39 09 657 A1 offenbart. Er besteht im unverformten Zustand aus zwei bogenförmigen Abschnitten, so daß das Schlauchlumen die Querschnittsform einer bikonvexen Linse hat. Die Muffen selbst sind kreiszylindrisch. Durch diese Querschnittsform des Pumpenschlauches wird die Kraft, welche zum okklusiven Verschluß des Schlauches durch die Pumpenschieber der Peristaltikpumpe aufzuwenden ist, verringert. Von den Verbindungsstellen der beiden bogenförmigen Schlauchwandabschnitte stehen Stege nach außen ab, deren Stärke etwa der Summe der Stärke der beiden bogenförmigen Abschnitte entspricht. Die Stege vermindern die Materialbelastung, weil ein Teil der von außen her auf den Schlauch einwirkenden Okklusionskraft von diesen aufgenommen wird. Toleranzen der Infusionspumpe bei der Bemessung der auf den Schlauch einwirkenden Kraft werden eliminiert.

EP 0024 431 offenbart eine peristaltische Pumpe, deren Pumpelement aus zwei Bahnen gebildet ist, die bis auf einen zentralen Längsteil flächig miteinander verschweißt sind. Der zentrale Längsteil formt eine Pumpenkammer, deren Unterseite mit mehreren topfartigen flexiblen Gebilden ausgestattet ist, deren Volumen zur Erzielung des Pumpeffektes von Kolben verändert wird. An die beiden axialen Enden des Längsteiles sind ein Einlaß und ein Auslaßschlauch angeschlossen. An einem Längsrand des bahnförmigen Pumpelementes befinden sich im Bereich seiner Enden ein Langloch und ein Kreisloch, durch die beim Auflegen des Pumpelementes auf eine Stützfläche am Pumpengehäuse kreiszylindrische Stifte, die von der Stützfläche abstehen, hindurchpassen. Der Zusammengriff des Kreisloches mit dem einen Stift soll die richtige Orientierung des Pumpelementes vorgeben. Für einen Pumpenschlauch mit endseitigen Muffen ist ein solcher "Druckknopf" nicht brauchbar, weil es bei einem solchen Pumpenschlauch darauf ankommt, ihn hängend zu fixieren, ohne daß sein Fördervolumen durch Abmessungsveränderungen verfälscht wird.

Der Erfindung liegt die Aufgabe zugrunde, einen Pumpenschlauch zu schaffen, der mit in bezug auf die Saugseite und die Druckseite der Pumpe korrekter Fließrichtung problemlos in der Pumpe befestigbar ist und sich durch gleichmäßiges Förderverhalten auszeichnet.

Diese Aufgabe wird erfindungsgemäß dadurch gelöst, daß an jeder Muffe eine Aufhängevorrichtung in Form von zwei symmetrisch zur Längsachse des Schlauches nach entgegengesetzten Seiten gerichteten Laschen angeordnet ist, in denen je eine Lochöse ausgebildet ist,
daß die Lochösen in den Laschen am saugseitigen Ende des Schlauches eine andere Form und/ oder Größe als die Lochösen in den Laschen am druckseitigen Ende des Schlauches haben,
und daß die Lochösen auf angepaßte Haltestifte der Pumpe aufsteckbar sind.

Die Aufhängevorrichtung und die Haltestifte, welche in Fließrichtung vor der Pumpe (saugseitig) liegen, unterscheiden sich deutlich von jenen, welche in Fließrichtung hinter der Pumpe (druckseitig) angeordnet sind, so daß die Fließrichtung, nach der der Schlauch zu orientieren ist, für den Anwender nicht nur eindeutig erkennbar ist, sondern dieser zusätzlich durch die unterschiedlichen Passungen der Teile zu richtiger Handhabung gezwungen wird. Eine einfache Geometrie der Lochösen und der angepaßten Haltestifte erleichtert sowohl die Erkennbarkeit der Zusammengehörigkeit der geräteseitigen und schlauchseitigen Fixierelemente als auch die Montage des Pumpenschlauches in der Pumpe. Durch Aufstecken der Lochösen auf die angepaßten Haltestifte der Pumpe ist eine einfache korrekte Fixierung des Pumpenschlauches möglich. Durch einfaches Aufhängen der Lochösen auf die angepaßten Kaltestifte der Pumpe wird der Pumpenschlauch mit definiertem Abstand der saugseitigen Muffe von der druckseitigen Muffe in die Pumpe eingelegt. Das Längenmaß ist damit werkzeuggebunden und nicht von der Montage abhängig. Da der Abstand der saugseitigen und der druckseitigen Fixierelemente weitgehend frei von Herstellungs- und Montagetoleranzen ist, wird eine undefinierte Schlauchdehnung des Pumpsegmentes vermieden und damit eine hohe Fördergenauigkeit erreicht. Die Pumpe arbeitet in einem engen Toleranzbereich praktisch schwankungsfrei und mit vorgegebener Förderleistung, da der Pumpenschlauch weder gedehnt noch gestaucht wird. Ein weiterer Vorteil der Spannungsfreiheit des Schlauches besteht in der Vermeidung von Materialschädigungen und einer Verbesserung des Rückstellverhaltens. Die Standzeit des Pumpenschlauches wird verlängert und die Konstanz des Fördervolumens über die Infusionszeit erhöht.

In vorteilhafter Ausgestaltung der Erfindung ist vorgesehen, daß die Laschen des saugseitigen Endes gemeinsam eine Pfeilspitze formen, die gegen das druckseitige Ende weist und daß die Laschen des druckseitigen Endes rechtwinklige Form haben. Die Laschenpaare erleichtern die Handhabung des Pumpenschlauches bei der Einlegung in die Pumpe und sorgen für sicheren Halt. Durch die Pfeilspitzenform der Laschen an der Saugseite und die abweichende Form der Laschen an der Druckseite wird die Fließrichtung eindeutig und unverwechselbar kenntlich gemacht.

Als weitere Hilfe zur Vermeidung einer Fehlorientierung des Pumpenschlauches ist vorgesehen, daß die Mittelpunkte der symmetrisch zur Schlauchlängsachse angeordneten Lochösen am saugseitigen Ende außerhalb der Flucht der Mittelpunkte der symmetrisch zur Schlauchlängsachse angeordneten Lochöffnungen am druckseitigen Ende liegen.

Die Lochösen in den Laschen am saugseitigen Ende können kreisförmig sein, während die Lochösen in den Laschen am druckseitigen Ende rechteckig sind und ihre Längsachsen parallel zur Schlauchlängsachse verlaufen. Die angepaßten Haltestifte haben entsprechend am saugseitigen Ende des Pumpenkanals angepaßten kreisförmigen Querschnitt und am druckseitigen Ende des Pumpenkanals angepaßten rechteckigen Querschnitt. Um ein gewaltsames Aufstecken der kreisförmigen Löcher auf die rechteckigen Haltestifte zu verhindern, sind die rechteckigen Haltestifte wesentlich breiter als der Durchmesser der kreisförmigen Lochösen. Vorzugsweise sind die Haltestifte mit kreisförmigem Querschnitt schräg nach oben gerichtet, so daß die Laschen nicht nach vorne von den Haltestiften abrutschen können. Die unteren rechteckigen Haltestifte können im wesentlichen waagerecht orientiert sein, wobei die Schlauchhalterung durch Reibungsschluß erreicht wird. Der gegenseitige Abstand der Haltestiftpaare entspricht im wesentlichen dem Abstand der Lochösenpaare.

Die Laschen sind bevorzugt einstückig an die Muffen angeformt. Sie liegen in gemeinsamer Ebene und überragen die axialen Enden der Muffen nicht.

In jede Muffe ist ein rohrförmiges Fixierstück einrastend eingepaßt, in das ein Zuleitungsschlauchende bzw. ein Ableitungsschlauchende der Infusionsleitung festsitzend eingeschoben ist. Die rohrförmigen Fixierstücke sind starre Kunststoffteile, die die aus dem flexiblen Schlauchmaterial bestehenden Muffen versteifen und damit den korrekten Sitz der ebenfalls an sich flexiblen Laschen an den Haltestiften verbessern.

In der Zeichnung ist ein Ausführungsbeispiel der Erfindung schematisch dargestellt. Es zeigt:
Figur 1 eine Draufsicht auf einen Pumpenschlauch für eine peristaltische Pumpe,

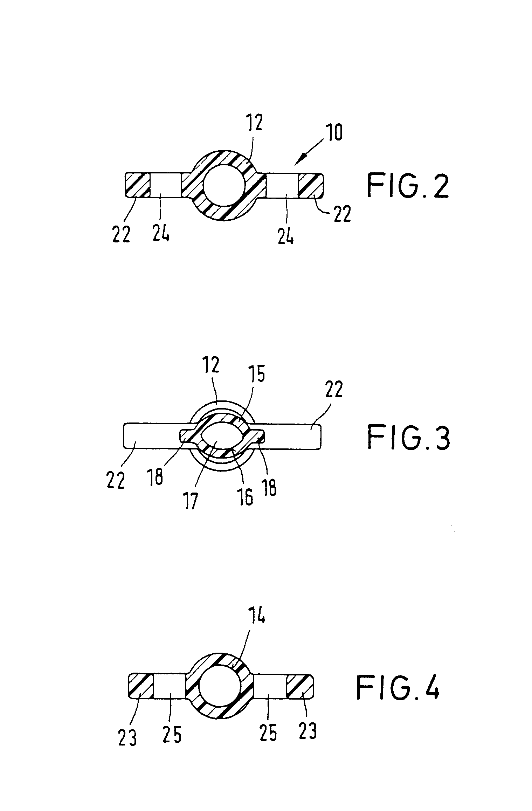

Figur 2 einen Schnitt längs der Linie 11-11 in Figur 1,
Figur 3 einen Schnitt längs der Linie 111-111 in Figur 1,
Figur 4 einen Schnitt längs der Linie IV-IV in Figur 1, und
Figur 5 einen Längsschnitt durch einen Teil einer peristaltischen Pumpe mit eingebautem Pumpenschlauch.

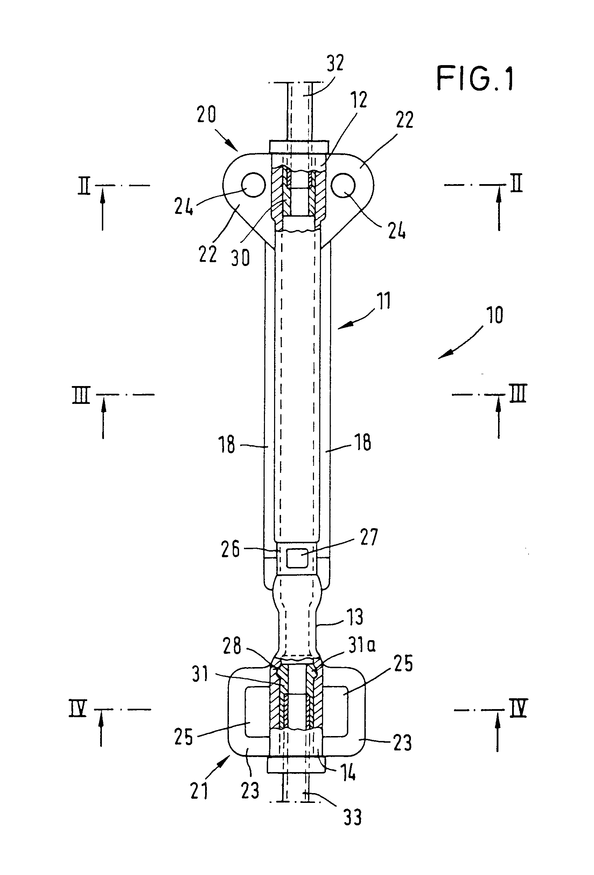

Der Pumpenschlauch 10 gemäß Figur 1 besteht aus hochelastischem Material, z.B. Silikon. Er weist einen langgestreckten Mittelteil 11 auf, an dessen oberes Ende eine kreiszylindrische Muffe 12 angeformt ist und dessen unteres Ende über einen Zwischenabschnitt 13 mit einer kreiszylindrischen Muffe 14 versehen ist. Der Mittelteil 11 des Schlauches 10 weist im Querschnitt zwei bogenförmige Abschnitte 15, 16 auf, die ein Schlauchlumen 17 derart umschließen, daß es die Querschnittsform einer bikonvexen Linse hat. An die beiden Längsränder des Schlauches 10 in der Ebene der längeren Achse seines Querschnittes schließen sich nach außen weisende Stege 18 an. Die Stärke eines jeden Steges 18 entspricht etwa der Summe der Wandstärken der beiden bogenförmigen Abschnitte 15 und 16.

Jede Muffe 12, 14 ist mit einer Aufhängevorrichtung 20, 21 versehen, von denen die eine in montiertem Zustand saugseitig und die andere druckseitig angeordnet sein soll. Die eine Aufhängevorrichtung 20 besteht aus zwei identischen Laschen 22, die in gemeinsamer Ebene symmetrisch zur Längsachse des Schlauches 10 nach entgegengesetzten Seiten gerichtet und einstückig an den Körper des Schlauches 10 angeformt sind. Jede Lasche 22 ist dreieckig mit abgerundeter Ecke und die beiden Laschen 22 sind so orientiert, daß eine gegen das andere Ende des Schlauches 10 weisende Pfeilspitze entsteht. Mit dem Öffnungsrand der Muffe 12 schließen die beiden Laschen 22 bündig ab. Das Pfeilspitzenende der beiden Laschen 22 geht in die beiden Stege 18 des Mittelteiles 11 des Schlauches 10 über. Die Dicke der Laschen 22 ist jeweils größer als diejenige der Stege 18 (Figur 3). In jeder Lasche 22 ist eine Lochöse 24 in Form einer kreisrunden Durchbrechung ausgebildet. Die beiden Lochösen 24 sind zur Schlauchlängsachse symmetrisch angeordnet und liegen mit ihren Mittelpunkten auf einer gemeinsamen Linie, die zur Schlauchlängsachse senkrecht verläuft.

Die Aufhängevorrichtung 21 am anderen Ende des Schlauches 10 besteht aus zwei an die Muffe 14 angeformten und symmetrisch zur Längsachse des Schlauches 10 nach entgegengesetzen Seiten gerichteten identischen Laschen 23, die in gemeinsamer Ebene und in der Ebene der Laschen 22 liegen. Die beiden Laschen 23 haben rechteckige Form mit abgerundeten äußeren Ecken. Sie sind jeweils von einer rechteckigen Lochöse 25 durchbrochen. Die Längsachsen der Laschen 23 und der Lochösen 25 verlaufen parallel zur Längsachse des Schlauches 10. Die Mittelpunkte der Lochösen 25 sind zur Schlauchlängsachse symmetrisch angeordnet und liegen auf einer gemeinsamen Linie, die zur Schlauchlängsachse senkrecht verläuft. In bezug auf die Mittelpunkte der Lochösen 24 liegen die Mittelpunkte der Lochösen 25 nicht in gerader Flucht unter diesen, sondern sie sind symmetrisch etwas nach innen versetzt. Die rechteckigen Lochösen 25 schließen sich direkt an die Außenfläche der Muffe 14 an.

In der Nähe des unteren Endes des Mittelteiles 11 mit linsenförmigem Querschnitt ist die Wandstärke des Schlauches 10 reduziert, so daß ein Längenabschnitt entsteht, der als Druckmeßzone 26 benutzt werden kann, an die ein den Innendruck messender Drucksensor von außen angesetzt werden kann. Eine nach außen gerichtete Warze 27 an der Druckmeßzone 26 verhindert Beschädigungen der Schlauchwandung unter dem Einfluß des Drucksensors.

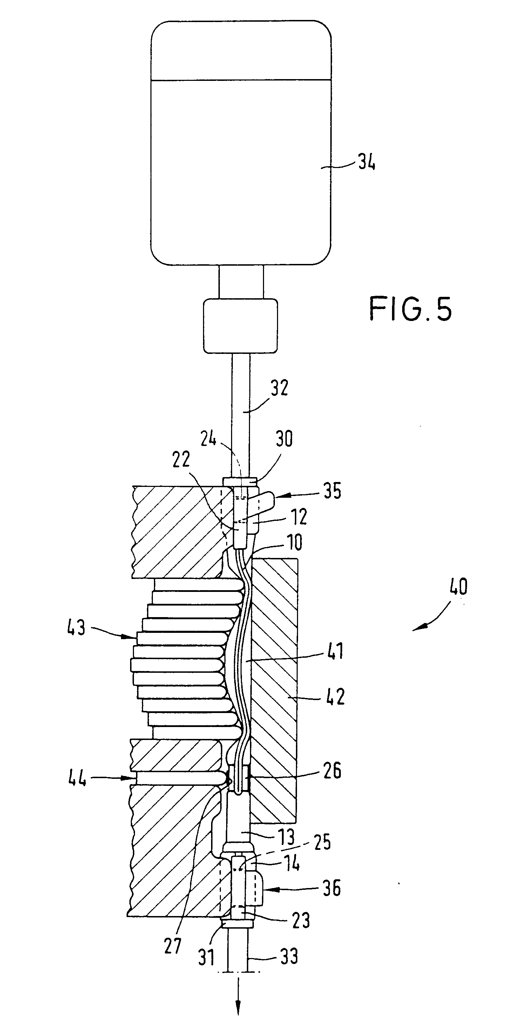

In jede Muffe 12, 14 ist ein rohrförmiges Fixierstück 30,31 eingepaßt, dessen kreiszylindrischer Kanal der Befestigung eines eingeschobenen Zuleitungsschlauches 32 bzw. eines Ableitungsschlauches 33 dient, die Teil einer Infusionsleitung sind, welche einen Infusionsflüssigkeitsbehälter 34 mit einem Patienten verbindet (Figur 5). Das untere Fixierstück 31 rastet mit einem äußeren Ringbund 31a a in eine Ringrille 28 am Übergang des Schlauchlumens zwischen der Muffe 14 und dem Mittelteil 11 ein. Der Zuleitungsschlauch 32 und der Ableitungsschlauch 33 sind zweckmäßigerweise in die Fixierstücke 30, 31 eingeklebt oder eingeschweißt.

Der Pumpenschlauch 10 wird werkseitig mit dem Zuleitungsschlauch 32 und dem Ableitungsschlauch 33 ausgerüstet und der Anwender baut die bis zu 2 m lange Infusionsleitung in eine peristaltische Pumpe 40 ein. Dabei kommt es darauf an, daß der Zuleitungsschlauch 32 sich am saugseitigen Ende der Pumpe 40 befindet und der Ableitungsschlauch 33 dem druckseitigen Ende der Pumpe 40 zugeordnet ist, damit die Fließrichtung von dem Infusionsflüssigkeitsbehälter 34 zum Patienten gewährleistet ist. Die Fließrichtung erkennt der Benutzer bei der Montage des Pumpenschlauches 10 in der peristaltischen Pumpe 40 zunächst an der Weisungsrichtung der Pfeilspitze der beiden Laschen 22. Außerdem wird er zu einer korrekten Montage dadurch gezwungen, daß die Lochösen 24 und 25 nur auf angepaßte Haltestifte der Pumpe 40 aufsteckbar sind, d.h., bei falscher Orientierung des Pumpenschlauches 10 und Verwechslung der Lochösen 24 bzw. 25 lassen sich diese nicht zwanglos auf die Haltestifte aufstecken. Den kreisförmigen Lochösen 24, die dem saugseitigen Ende des Schlauches 10 zugeordnet sind und die sich im montierten Zustand oben befinden, sind zwei Haltestifte 35 mit kreisförmigem Querschnitt zugeordnet, die schräg nach oben weisen und deren Durchmesser dem Durchmesser der Lochösen 24 angepaßt ist, so daß diese praktisch ohne Spiel auf die Haltestifte 35 aufsteckbar sind.

Das unten befindliche druckseitige Ende des Pumpenschlauches 10 wird mit Hilfe von zwei an der Pumpe 40 vorgesehenen Haltestiften 36 mit rechteckigem Querschnitt fixiert, wobei die senkrechten Abstände der Lochösen 24, 25 und der Haltestifte 35, 36 so gewählt sind, daß der Schlauch 10 definiert gespannt wird, d.h. das Längenmaß ist werkzeuggebunden und nicht montageabhängig und die Förderrate ist im engen Toleranzbereich konstant. Die Haltestifte 36 bilden Stege, die rechtwinklig von einer senkrechten Fläche der Pumpe 40 nach außen abstehen. Ihr Querschnitt ist so bemessen, daß er in die rechteckigen Lochösen 25 paßt. Die senkrechte Breite der Haltestifte 36 ist wesentlich größer als der Durchmesser der kreisförmigen Löcher 24, damit die runden Löcher nicht gewaltsam auf die rechteckigen Haltestifte 36 aufgesteckt werden können. Entsprechend den Verhältnissen bei den Lochösen 24 und 25 sind auch die Abstände der Haltestifte 35 voneinander anders als die Abstände der Haltestifte 36 voneinander, so daß auch hierdurch eine zusätzliche Verhinderung eines falsch orientierten Einbaus des Pumpenschlauches 10 in die Pumpe 40 erreicht wird.

Der zwischen den Haltestiften 35 und 36 hängende Pumpenschlauch 10 verläuft in einem geraden Kanal 41 der Pumpe 40 linear. Er ist an einer Seite von einem Gegenlager 42 abgestützt, das zur Montage und Demontage des Pumpenschlauches 10 von dem Kanal 41 entfernbar ist. Auf der gegenüberliegenden Seite wirken mehrere Pumpenschieber 43 auf den Mittelteil 11 des Schlauches 10 ein. Die Pumpenschieber 43 führen Hubbewegungen gegen den Mittelteil 11 aus und quetschen ihn peristaltisch ab, wodurch sich eine Förderung von Infusionsflüssigkeit aus dem Infusionsflüssigkeitsbehälter 34 durch den Pumpenschlauch 10 hindurch zu dem Patienten ergibt. Die Pumpenschieber 43 können von einer Nockenwelle angetrieben sein. Gegen die Druckmeßzone 26 des Pumpenschlauches 10 ist ein Drucksensor 44 angesetzt.

## Patentansprüche

1. Pumpenschlauch für eine peristaltische Pumpe (40), der an seinem saugseitigen und an seinem druckseitigen Ende mit einer einstückig angeformten Muffe (12,14) versehen und durch Halteteile an der Pumpe (40) hängend befestigbar ist, dadurch gekennzeichnet, daß
an jeder Muffe (12,14) eine Aufhängevorrichtung (20,21) in Form von zwei symmetrisch zur Längsachse des Schlauches (10) nach entgegengesetzten Seiten gerichteten Laschen (22,23) angeordnet ist, in denen je eine Lochöse (24,25) ausgebildet ist,
daß die Lochösen (24) in den Laschen (22) am saugseitigen Ende des Schlauches (10) eine andere Form und/ oder Größe als die Lochösen (25) in den Laschen (23) am druckseitigen Ende des Schlauches (10) haben, und
daß die Lochösen (24,25) auf angepaßte Haltestifte (35,36) der Pumpe (40) aufsteckbar sind.

2. Pumpenschlauch nach Anspruch 1, dadurch gekennzeichnet, daß die Laschen (22) des saugseitigen Endes gemeinsam eine Pfeilspitze formen, die gegen das druckseitige Ende weist, und daß die Laschen (23) des druckseitigen Endes rechtwinklige Form haben.

3. Pumpenschlauch nach Anspruch 1 oder 2, dadurch gekennzeichnet, daß die Mittelpunkte der symmetrisch zur Schlauchlängsachse angeordneten Lochösen (24) am saugseitigen Ende außerhalb der Flucht der Mittelpunkte der symmetrisch zur Schlauchlängsachse angeordneten Lochöffnungen (25) am druckseitigen Ende liegen.

4. Pumpenschlauch nach einem der Ansprüche 1 bis 3, dadurch gekennzeichnet, daß die Lochösen (24) in den Laschen (22) am saugseitigen Ende kreisförmig sind, und daß die Lochösen (25) in den Laschen (23) am druckseitigen Ende rechteckig sind und die Längsachsen der Lochösen (25) parallel zur Schlauchlängsachse verlaufen.

5. Pumpenschlauch nach einem der Ansprüche 1 bis 4, dadurch gekennzeichnet, daß die Laschen (22,23) einstückig an die Muffen (12,14) angeformt sind.

6. Pumpenschlauch nach einem der Ansprüche 1 bis 5, dadurch gekennzeichnet, daß in jede Muffe (12,14) ein rohrförmiges Fixierstück (30,31) eingepaßt ist, in das ein Zuleitungsschlauchende (32) bzw. ein Ableitungsschlauchende (33) festsitzend eingeschoben ist.

7. Pumpenschlauch nach einem der Ansprüche 1 bis 6, dadurch gekennzeichnet, daß der Schlauch (10) linsenförmiges äußeres und inneres Querschnittsprofil hat und daß die Muffen (12,14) außen und innen kreiszylindrisch gestaltet sind.
